(19) Europäisches Patentamt
European Patent Office
Office européen des brevets

(11) Veröffentlichungsnummer: **0 024 272**
**B1**

(12) # EUROPÄISCHE PATENTSCHRIFT

(45) Veröffentlichungstag der Patentschrift: **08.12.82**

(51) Int. Cl.³: **C 07 D 243/16**, A 61 K 31/55

(21) Anmeldenummer: **78101586.2**

(22) Anmeldetag: **05.12.78**

(54) **1,4-Benzodiazepinderivate und ihre Salze; Verfahren zu ihrer Herstellung und diese Verbindungen enthaltende Arzneipräparate.**

(30) Priorität: **05.12.77 DE 2754112**

(43) Veröffentlichungstag der Anmeldung:
**04.03.81 Patentblatt 81/9**

(45) Bekanntmachung des Hinweises auf die Patenterteilung:
**08.12.82 Patentblatt 82/49**

(84) Benannte Vertragsstaaten:
**BE CH DE FR GB IT LU NL SE**

(56) Entgegenhaltungen:
**DE-A-2 353 187**
**FR-A-2 183 735**
**GB-A-1 517 164**

**Die Akte enthält technische Angaben, die nach dem Eingang der Anmeldung eingereicht wurden und die nicht in dieser Patentschrift enthalten sind.**

(73) Patentinhaber: **Kali-Chemie Pharma GmbH, Hans-Böckler-Allee 20 Postfach 220, D-3000 Hannover 1 (DE)**

(72) Erfinder: **Liepmann, Hans, Dipl-Chem. Dr. rer. nat., Auf dem Emmerberge 17, D-3000 Hannover (DE)**
Erfinder: **Hüschens, Rolf,Dipl.-Chem. Dr. rer. nat., Matthäikirchstrasse 25, D-3000 Hannover 81 (DE)**
Erfinder: **Milkowski, Wolfgang Dipl.-Chem. Dr. rer. nat, Fasanenweg 13, D-3167 Burgdorf (DE)**
Erfinder: **Zeugner, Horst Dipl.-Chem., Dr. rer.nat., Havelweg 10, D-3000 Hannover 73 (DE)**
Erfinder: **Budden, Renke Dr. med.vet., Rodewaldestrasse 18, D-3000 Hannover (DE)**
Erfinder: **Bahlsen, Jacqueline Dr. med.vet., Lindemannalle 15, D-3000 Hannover 1 (DE)**

(74) Vertreter: **Lauer, Dieter, Dr. et al, c/o Kali-Chemie Aktiengesellschaft Postfach 220, D-3000 Hannover 1 (DE)**

EP 0 024 272 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# 1,4-Benzodiazepinderivate und ihre Salze; Verfahren zu ihrer Herstellung und diese Verbindungen enthaltende Arzneipräparate

Die Erfindung betrifft 1,4-Benzodiazepinderivate der Formel (I)

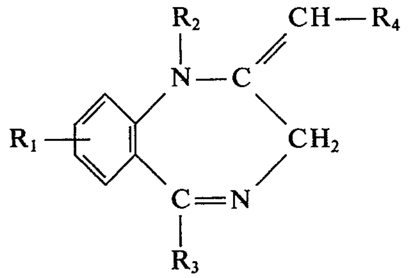

(I)

in welcher

$R_1$ Wasserstoff, Halogen oder Nitro,
$R_2$ Alkyl mit 1—6 Kohlenstoffatomen,
$R_3$ Phenyl, Halogenphenyl oder Trifluormethylphenyl und
$R_4$ Cyano oder Carbamoyl bedeuten,

sowie deren Säureadditionssalze.

Der Alkylrest $R_2$ bedeutet einen geraden oder verzweigten Kohlenwasserstoffrest mit 1 bis 6, vorzugsweise 1 bis 3 Kohlenstoffatomen, vorzugsweise Methyl, Äthyl, n-Propyl oder Isopropyl, aber auch n-Butyl, Isobutyl, sec.-Butyl, tert.-Butyl, n-Amyl, Isoamyl, 2-Methylbutyl-(1), Pentyl-(1), Pentyl-(2), 3-Methylbutyl-(2), tert.-Amyl, n-Hexyl oder Isohexyl. Der Ausdruck »Halogen« bedeutet die vier Halogenatome Chlor, Brom, Jod und Fluor.

Bevorzugte Gruppen von Verbindungen der Formel (I) sind solche, in welcher $R_1$ in 7-Stellung des Benzodiazepingerüstes steht und/oder $R_2$ Methyl bedeutet und/oder $R_3$ Phenyl, o-Halogenphenyl oder o-Trifluormethylphenyl ist.

Die Verbindungen der Formel (I) können dadurch hergestellt werden, daß man eine Verbindung der Formel (II)

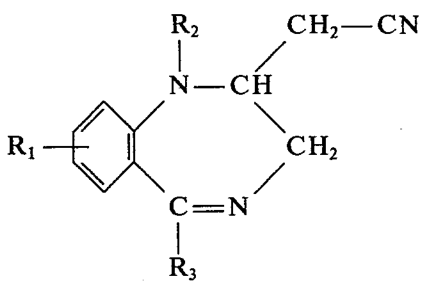

(II)

in welcher $R_1$, $R_2$ und $R_3$ die oben angegebene Bedeutung haben, mit Chrom(VI)-oxid in stark saurer Lösung zu einer Verbindung der Formel Ia

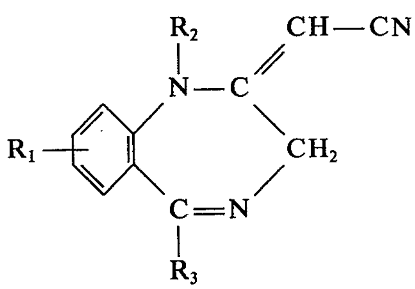

(Ia)

in welcher $R_1$, $R_2$ und $R_3$ die oben angegebene Bedeutung haben, oxydiert und gegebenenfalls durch saure Hydrolyse des Cyanorestes zum Carbamoylrest in die Verbindung der Formel (Ib)

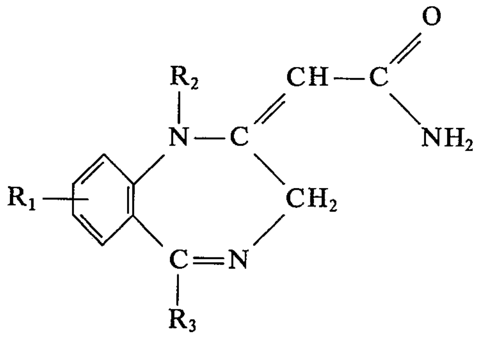

(Ib)

in welcher $R_1$, $R_2$ und $R_3$ die oben angegebene Bedeutung haben, überführt. Die dabei erhaltenen freien Basen können gewünschtenfalls in die Säureadditionssalze bzw. die erhaltenen Säureadditionssalze in die freien Basen übergeführt werden.

Das erfindungsgemäße Verfahren ist in zweierlei Hinsicht überraschend; es war weder die oxydative Überführung der 2-Cyanomethylgruppe in die 2-Cyanomethylengruppe zu erwarten noch deren Hydrolyse in die 2-Carbamoylmethylengruppe in guter Ausbeute vorauszusehen.

Aus der DE-OS 2 221 536 ist bekannt, daß ganz allgemein 1,4-Benzodiazepinderivate, welche in 2-Stellung eine substituierte Methylgruppe besitzen, durch Oxydationsmittel unter Abbau der Seitenkette in 2-Stellung des 1,4-Benzodiazepingerüstes zu 1,4-Benzodiazepin-2-on-derivaten oxydiert werden. Als Oxydationsmittel können dabei Kaliumpermanganat, Mangandioxid, Chromtrioxid oder Dichromatsalze eingesetzt werden, wobei im allgemeinen Kaliumpermanganat bevorzugt wird. Auch die 2-Cyanomethylverbindungen der Formel II lassen sich in wäßriger Salzsäure mit Kaliumpermanganat mit guter Ausbeute in die entsprechenden 1,4-Benzodiazepin-2-onderivate überführen, wobei jedoch ungesättigte Nitrile der Formel I nur in untergeordnetem Maße nachgewiesen werden können.

Es wurde nun gefunden, daß bei Behandlung der 2-Cyanomethylverbindungen der Formel (II) mit Chrom-IV-oxid unter geeigneten Bedingungen die ungesättigten Nitrile der Formel (Ia) in guter Ausbeute erhalten werden. Dieses ist überraschend, da sich gezeigt hat, daß diese ungesättigten Nitrile ohne Schwierigkeiten mit Kaliumpermanganat in saurer Lösung zu den 1,4-Benzodiazepin-2-onderivaten oxydiert werden können.

Das Verfahren wird unter Verwendung von Chrom(VI)-oxid in saurer Lösung vorgenommen; es können auch andere Chromverbindungen herangezogen werden, die in saurer Lösung zur Bildung von Chromsäure geeignet sind, insbesondere Alkalichromat- oder -dichromatsalze. Der für die Oxydation günstigste pH-Wert wird vorzugsweise mit anorganischen Säuren, beispielsweise Salzsäure oder Schwefelsäure, eingestellt.

Die Oxydationsmittel werden vorzugsweise in Lösung oder als fein verteilte Suspension in einer Lösung des Ausgangsstoffes der Formel (II) eingesetzt.

Zu den vielen geeigneten inerten Lösungsmitteln zählen beispielsweise Dimethylformamid, Kohlenwasserstoffe, wie Benzol, Toluol, Xylol, weiter tertiäre organische Basen, wie Pyridin, halogenierte Kohlenwasserstoffe, wie Methylenchlorid oder Chloroform, und niedere Alkansäuren, wie Essigsäure oder Propionsäure.

Anstelle der genannten Lösungsmittel können auch Gemische von solchen Lösungsmitteln eingesetzt werden, oder man kann die genannten Lösungsmittel im Gemisch mit Wasser in einem Einphasen- oder Zweiphasensystem verwenden.

Die Reaktionstemperatur liegt im vorliegenden Verfahren im allgemeinen zwischen 0 und 50°C, unter Umständen können aber auch höhere Temperaturen erforderlich werden.

So werden beispielsweise gute Ausbeuten an ungesättigten Nitrilen der Formel (Ia) erhalten, wenn man eine salz- oder schwefelsaure Lösung von Chrom(VI)-oxid einsetzt und unter Verwendung von Eisessig als Lösungsmittel bei Temperaturen zwischen 10 bis 20°C arbeitet.

Aus den 2-Cyanomethylen-1,4-benzodiazepinen der Formel (Ia), welche selbst eine gewisse psychopharmakologische Wirkung zeigen, können durch saure Hydrolyse die 2-Carbamoylmethylen-1,4-benzodiazepine I gewonnen werden, welche aufgrund der im Tierversuch erzielten Ergebnisse besonders als Psychosedativa in der Therapie geeignet sind.

Es wurde nämlich gefunden, daß sich die 2-Cyanomethylengruppe in den Verbindungen der Formel (Ia) durch saure Hydrolyse in die 2-Carbamoylmethylengruppe überführen läßt, ohne daß die exocyclische Doppelbindung angegriffen bzw. isomerisiert wird. Es war nicht ohne weiteres vorauszusehen, daß diese Doppelbindung unter den erforderlichen Reaktionsbedingungen nicht in den Benzodiazepinring unter Bildung von 1H-1,4-Benzodiazepinderivaten isomerisiert wird.

Die saure Hydrolyse der Cyangruppe kann in üblicher Weise durch starke Säuren wie Chlorwasserstoffsäure, Bromwasserstoffsäure, Schwefelsäure, aber auch mit Polyphosphorsäure

oder Bortrifluorid durchgeführt werden, wobei die Reaktion in An- und Abwesenheit eines inerten Lösungsmittels durchgeführt werden kann. Als Lösungsmittel eignen sich beispielsweise niedere Alkansäuren, insbesondere Essigsäure. Die zweckmäßige Reaktionstemperatur liegt im allgemeinen zwischen 0 und 100° C und richtet sich nach der zur Hydrolyse verwendeten Säure. Im vorliegenden Verfahren wird die Hydrolyse der Cyangruppe vorzugsweise in konzentrierter Schwefelsäure bei Temperaturen von 30 bis 60° C vorgenommen.

Die Ausgangsverbindungen der Formel (II) sind bekannt. Die Herstellung derselben erfolgt beispielsweise entsprechend dem in der DE-A-2 221 558 beschriebenen Verfahren, gemäß welchem Acyldiamine der allgemeinen Formel (III)

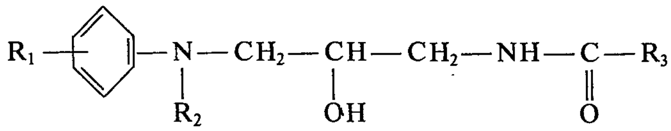

(III)

in welcher $R_1$, $R_2$ und $R_3$ die oben angegebene Bedeutung haben, mit Phosphoroxychlorid bei Rückflußtemperatur cyclisiert und anschließend durch Umsetzung mit KCN in die Verbindungen der Formel (II) übergeführt werden.

Es wird besonders darauf hingewiesen, daß man auch ohne Isolierung und Reinigung des ungesättigten Nitrils der Formel (Ia) die Reaktion bis zu den 2-Carbamoylmethylen-1,4-benzodiazepinen durchführen kann.

Die Aufarbeitung der Reaktionsprodukte sowie die Isolierung und Reinigung der 2-Carbamoylmethylen-1,4-benzodiazepine der Formel (Ib) erfolgt in an sich bekannter Weise. Man kann beispielsweise die Reaktionslösung durch Zugabe von Eis zersetzen und das Rohprodukt mit einem geeigneten organischen Lösungsmittel, wie beispielsweise Methylenchlorid, Chloroform, Äther, Essigester, Toluol oder Benzol, aus der wäßrigen Phase extrahieren. Zur weiteren Reinigung kann man das Lösungsmittel abdestillieren und den Rückstand aus einem geeigneten Lösungsmittel oder Lösungsmittelgemisch umkristallisieren. Dafür eignen sich beispielsweise Toluol, Benzol, Essigester, Äthanol; die Auswahl bietet dem Fachmann bei Berücksichtigung der Lösungsmitteleigenschaften keinerlei Schwierigkeiten.

Die nach den erfindungsgemäßen Verfahren erhaltenen Verbindungen der allgemeinen Formel (I) werden gewünschtenfalls in üblicher Weise in ihre Additionssalze mit anorganischen und organischen Säuren übergeführt. Dazu versetzt man beispielsweise eine Lösung einer Verbindung der Formel (I) in einem organischen Lösungsmittel mit der als Salzkomponente gewünschten Säure. Vorzugsweise wählt man für die Umsetzung organische Lösungsmittel, in denen das entstehende Salz schwer löslich ist, damit es durch Filtration abgetrennt werden kann. Solche Lösungsmittel sind beispielsweise Äthanol, Isopropanol, Äther, Aceton, Methyläthylketon bzw. Lösungsmittelgemische wie Aceton/Äther, Aceton/Äthanol oder Äthanol/Äther.

Die Carbamoyl-Verbindungen der Formel (Ib) und ihre Additionssalze mit pharmakologisch annehmbaren anorganischen und organischen Säuren haben wertvolle pharmakologische Eigenschaften. Sie weisen zentraldämpfende, krampflösende, muskelentspannende, angstvermindernde und sedative Wirkungen auf. Außerdem besitzen sie eine geringe Toxizität.

Aus dem französischen Patent Nr. 2 183 735 sind in 2-Stellung eine substituierte Methylgruppe tragende 1-Alkyl-5-phenyl-1,3-dihydro-2H-1,4-benzodiazepine mit psychopharmakologischen, z. B. antikonvulsiven und antiaggressiven Eigenschaften bekannt.

## Beschreibung der pharmakologischen Untersuchungsmethoden

### 1. Akute Toxizität

Die akute 7-Tage-Toxizität wird nach einmaliger Applikation per os und intraperitoneal an der weißen nüchternen NMRI-Maus bestimmt. Die Berechnung der $LD_{50}$-Werte erfolgt über EDV durch eine Probitanalyse (Cavelli—Sforza, Gustav Fischer Verlag, (1964), Grundbegriffe der Biometrie, S. 153 ff.).

### 2. Prüfung auf antikovulsive Eigenschaften

#### Methode Pentetrazolkrampf

Die Untersuchungssubstanzen werden nach peroraler Verabreichung an Gruppen von je 6 Mäusen geprüft. 60 Minuten nach der Applikation der Versuchssubstanzen wird Pentetrazol subkutan in einer Dosis von 100 mg/kg injiziert. Das Auftreten von klonischen und tonischen Konvulsionen wird über eine Gesamtbeobachtungszeit von 45 Minuten kontrolliert. Die Schutzwirkung der Prüfsubstanzen gegen

Konvulsionen wird im Vergleich zu Kontrollversuchen bestimmt. Die effektive Dosis $ED_{50}$ wird aus den Probit log. Dosierungskurven berechnet. (Modifiziert nach J. E. Blum et al., Arzneimittel-Forsch. 23, 377 (1973).)

Die nach diesem Verfahren erhaltenen Ergebnisse zeigen die antikonvulsive Wirkung der Versuchssubstanzen und sind nach der Literatur ein wichtiges Kriterium für die Beurteilung der klinischen Wirksamkeit als Tranquilizer (A. Suria und E. Costa, Journal de Pharmacologie, Suppl. No. 1, 5, 94 (1974), und G. Zbinden und L. O. Randall, Advances in Pharmacol. 5, 257, (1967)).

### 3. Prüfung auf zentraldämpfende Eigenschaften (Beeinflussung der Seitenlage nach Hexobarbital)

Die Prüfsubstanz wird den Mäusen peroral appliziert. Nach 30 Minuten erhalten die Tiere zusätzlich eine i.v. Injektion von 64 mg/kg Hexobarbital. Der Zeitpunkt der Einnahme der Seitenlage wird festgestellt und die Dauer der Seitenlage mit einer nur mit Hexobarbital behandelten Kontrollgruppe verglichen. Als $ED_{50}$ ist eine Dosis definiert, bei der die Hälfte der Tiere eine um den Faktor 4 verlängerte Seitenlage gegenüber der Kontrollgruppe beibehält (J. W. Kemp, M. Tannhäuser und E. A. Swinyard, Arch. int. Pharmacodyn. 193 (1971), S. 37—47).

### 4. Prüfung auf angstlösende und antiaggressive Wirksamkeit

Hemmung der durch Isolation hervorgerufenen Aggressivität an der Maus

Vor dem Versuch werden die Mäuse 4 Wochen in strenger Isolation im Einzelkäfig gehalten. Nach dieser Zeit greifen die isoliert gehaltenen Mäuse nicht isoliert gehaltene Mäuse, die zugesetzt werden, spontan an. Die Prüfsubstanzen werden den isolierten Mäusen peroral verabreicht, und es wird nach 30 Minuten bzw. 60 Minuten die Dosis bestimmt, die zu einer 50%igen Reduktion des aggressiven Verhaltens führt (modifiziert nach Weischer und Opitz, Arch. int. Pharmacodyn. 195, 252 (1972)).

Die in dieser Versuchsanordnung erhaltenen Ergebnisse lassen gute Rückschlüsse auf die angst-, streß- und spannungslösenden Eigenschaften der Substanzen zu.

Folgende Verbindungen wurden nach den vorstehend beschriebenen Methoden untersucht:

Erfindungsgemäße Verbindungen:

Verbindung 1 (≙ Beispiel 1b):
7-Chlor-1-methyl-2-carbamoylmethylen-5-phenyl-1,3-dihydro-2H-1,4-benzodiazepin.

Verbindung 2 (≙ Beispiel 2b):
7-Chlor-1-methyl-2-carbamoylmethylen-5-(2'-chlorphenyl)-1,3-dihydro-2H-1,4-benzodiazepin.

Verbindung 3 (≙ Beispiel 5b):
7-Chlor-1-methyl-2-carbamoylmethylen-5-(2'-fluorphenyl)-1,3-dihydro-2H-1,4-benzodiazepin.

Verbindung 4 (≙ Beispiel 7b):
7-Chlor-1-methyl-2-carbamoylmethylen-5-(2'-trifluormethylphenyl)-1,3-dihydro-2H-1,4-benzodiazepin.

Verbindung 5 (≙ Beispiel 3b):
7-Brom-1-methyl-2-carbamoylmethylen-5-(2'-chlorphenyl)-1,3-dihydro-2H-1,4-benzodiazepin.

Verbindung 6 (≙ Beispiel 4b):
7-Brom-1-methyl-2-carbamoylmethylen-5-(2'-fluorphenyl)-1,3-dihydro-2H-1,4-benzodiazepin.

Verbindung 7 (≙ Beispiel 6b):
7-Nitro-1-methyl-2-carbamoylmethylen-5-(2'-chlorphenyl)-1,3-dihydro-2H-1,4-benzodiazepin.

Vergleichssubstanz:

Standard I:
Chlordiazepoxid = 7-Chlor-2-methyl-amino-5-phenyl-3H-1,4-benzodiazepin-4-oxid.

In Tabelle 1 sind die Werte für die Toxizität und die antikonvulsiven Wirkungen der oben angegebenen Verbindungen aufgeführt. Die Testergebnisse zeigen eine deutliche Überlegenheit der erfindungsgemäßen Substanzen.

Tabelle 1

| Substanz | $LD_{50}$ p.o. (mg/kg) | $LD_{50}$ i.p. (mg/kg) | Pentetrazolkrampf $ED_{50}$ (mg/kg) |
|---|---|---|---|
| 1 | >1470 | 228 | 2,4 |
| 2 | 1000 | 410 | 0,1 |
| 3 | >1470 | 572 | 0,4 |
| 4 | >1470 | 287 | 0,3 |
| 5 | >1470 | >1600 | 0,3 |
| 6 | >1470 | 574 | 0,4 |
| 7 | >1470 | n.b. | 1,0 |
| Stand. I | 903 | 300 | 5,0 |

In der Tabelle 2 sind die antiaggressiven und sedierenden Wirkungsqualitäten gegenübergestellt; auch hier zeigen die erfindungsgemäßen Substanzen eine bessere Wirksamkeit.

Tabelle 2

| Substanz | $LD_{50}$ i.p. (mg/kg) | Hexobarbitalschlafzeitverlängerung, $ED_{50}$ (mg/kg) | Isolierte Kampfmaus, $ED_{50}$ (mg/kg) 30′ | 60′ |
|---|---|---|---|---|
| 1 | 228 | 3,2 | 12,2 | 9,6 |
| 2 | 410 | 5 | 2,2 | 3,4 |
| 3 | 572 | 1,6 | 2,1 | 3,9 |
| 4 | 287 | 3,1 | 31,1 | 31,1 |
| 5 | >1600 | 1,3 | 5,4 | 4,6 |
| 6 | 574 | 2,6 | 4,8 | 5,7 |
| 7 | n. b. | 2,5 | n. b. | n. b. |
| Stand. I | 300 | 6 | 60 | 47 |

Die in den Tabellen 1 und 2 aufgezeigten Ergebnisse deuten auf gute angst- und streßlösende Eigenschaften der Substanzen hin. Gegenüber dem in der ärztlichen Praxis gebräuchlichen Chlordiazepoxid zeigen sie in den Testanordnungen eine wesentlich stärkere Wirksamkeit.

Die Wirkungsqualitäten, welche durch die ausgewählten pharmakologischen Standardversuche erfaßt wurden, charakterisieren die Verbindungen der Formel I als Wirkstoffe für Psychosedativa, die z. B. bei der Behandlung von Spannungs- und Erregungszuständen als Beruhigungsmittel und zur Behandlung von Neurosen verwendet werden können.

Zur Verwendung als Arzneistoffe können anstelle der freien Basen deren pharmazeutisch

annehmbare Säureadditionssalze eingesetzt werden, d. h. Salze mit solchen Säuren, deren Anionen bei den in Frage kommenden Dosierungen nicht toxisch sind. Ferner ist es von Vorteil, wenn die als Arzneistoffe zu verwendenden Salze gut kristallisierbar und nicht oder wenig hygroskopisch sind. Zur Salzbildung mit Verbindungen der Formel I können beispielsweise Chlorwasserstoffsäure, Bromwasserstoffsäure, Schwefelsäure, Phosphorsäure, Methansulfonsäure, Äthansulfonsäure, Benzolsulfonsäure, p-Toluolsulfonsäure, Citronensäure, Essigsäure, Milchsäure, Bernsteinsäure, Maleinsäure, Fumarsäure, Äpfelsäure, Weinsäure, Benzoesäure, Phenylessigsäure oder Mandelsäure verwendet werden.

Die Verbindungen der Formel Ib können in pharmazeutischen Gebrauchsformen verabreicht werden, welche 0,5 bis 200 mg Aktivsubstanz enthalten, wobei die Tagesdosis der zu behandelnden Spezies und den individuellen Erfordernissen angepaßt wird und vorzugsweise bei 1 bis 200 mg liegt. Parenterale Formulierungen werden im allgemeinen weniger Aktivsubstanz als Präparate zu oraler Verabreichung enthalten. Die Verbindungen der Formel Ib können allein oder in Kombination mit pharmazeutischen anwendbaren Trägermaterialien in vielen Dosierungsformen eingesetzt werden. Zum Beispiel kann man feste Präparate wie Tabletten, Kapseln, Pulver, Granulate, Emulsionen, Suspensionen und dergleichen für die orale Verabreichung einsetzen. Feste Präparate können einen anorganischen Trägerstoff wie Talkum oder einen organischen Trägerstoff wie Milchzucker oder Stärke enthalten. Zusätze wie Magnesiumstearat (ein Gleitmittel) können ebenfalls eingesetzt werden. Flüssige Präparate wie Lösungen, Suspensionen oder Emulsionen können die üblichen Verdünnungsmittel wie Wasser, Vaseline, Suspensionsmittel wie Polyoxyäthylenglykol und dergleichen enthalten. Es können auch zusätzlich andere Bestandteile zugegeben werden wie Konservierungsmittel, Stabilisierungsmittel und Netzmittel.

Die nachfolgenden Beispiele erläutern die Herstellung der neuen Verbindungen der Formel I:

Die Strukturen der neuen Verbindungen wurden durch spektroskopische Untersuchungen, insbesondere durch eine genaue Analyse der NMR-Spektren gesichert.

## Beispiel 1

### a) 7-Chlor-1-methyl-2-cyanomethylen-5-phenyl-1,3-dihydro-2H-1,4-benzodiazepin

Zu einer Lösung von 50 g 7-Chlor-1-methyl-2-cyanomethyl-5-phenyl-2,3-dihydro-1H-1,4-benzodiazepin (hergestellt nach dem Verfahren der DE-A-2 221 558 aus 7-Chlor-1-methyl-2-chlormethyl-5-phenyl-2,3-dihydro-1H-1,4-benzodiazepin und Kaliumcyanid) in 350 ml Eisessig tropft man 65 ml einer Lösung, bestehend aus 66,8 g Chromsäure und 57,5 ml konzentrierter Schwefelsäure in 250 ml Wasser, bei 13°C. Die Reaktionstemperatur von etwa 13°C wird anschließend noch 3 Stunden lang eingehalten. Danach gießt man das Reaktionsgemisch zur Aufarbeitung in 1 l Eiswasser und extrahiert das Rohprodukt mit Chloroform. Die organische Phase wird abgetrennt, nacheinander mit verdünnter Natronlauge und Wasser gewaschen und über Natriumsulfat getrocknet. Der nach Filtrieren und Eindampfen im Vakuum erhaltene Rückstand kristallisiert aus Äthanol. Man erhält 32,3 g 7-Chlor-1-methyl-2-cyanomethylen-5-phenyl-1,3-dihydro-2H-1,4-benzodiazepin vom Schmelzpunkt 225—227°C.

IR-Spektrum (KBr): 2198 $cm^{-1}$ (C=CH—CN).

### b) 7-Chlor-1-methyl-2-carbamoylmethylen-5-phenyl-1,3-dihydro-2H-1,4-benzodiazepin

15,2 g 7-Chlor-1-methyl-2-cyanomethylen-5-phenyl-1,3-dihydro-2H-1,4-benzodiazepin werden anschließend portionsweise unter Rühren in 80 ml konzentrierte Schwefelsäure eingetragen und die Suspension bis zur klaren Lösung allmählich auf 50°C erwärmt. Danach wird die Reaktionslösung eine Stunde bei 50°C gehalten. Nach dem Abkühlen wird zur Aufarbeitung auf Eis gegossen, die Schwefelsäure mit verdünnter Natronlauge neutralisiert und das Rohprodukt mit Methylenchlorid extrahiert. Die organische Phase wird abgetrennt, mit Wasser neutral gewaschen und über Natriumsulfat getrocknet. Der nach Filtrieren und Eindampfen im Vakuum erhaltene Rückstand wird aus Äthanol umkristallisiert. Man erhält 11 g 7-Chlor-1-methyl-2-carbamoylmethylen-5-phenyl-1,3-dihydro-2H-1,4-benzodiazepin vom Schmelzpunkt 179—181°C.

Summenformel: $C_{18}H_{16}CLN_3O$ · 0,4 Mol Äthanol.

Elementaranalyse:

| | | | | |
|---|---|---|---|---|
| ber. %: | C 65,76 | H 5,36 | N 12,15 | Cl 10,25 |
| gef. %: | C 65,64 | H 5,17 | N 11,85 | Cl 10,44 |

Beispiel 2

a) 7-Chlor-1-methyl-2-cyanomethylen-5-(2'-chlorphenyl)-1,3-dihydro-2H-1,4-benzodiazepin

Eine Lösung von 66,4 g 7-Chlor-1-methyl-2-cyanomethyl-5-(2'-chlorphenyl)-2,3-dihydro-1H-1,4-benzodiazepin in 250 ml Eisessig und 125 ml konzentrierter Salzsäure wird portionsweise bei einer Temperatur zwischen 10 bis 15°C mit 16 g Chromsäure versetzt. Nach einer Stunde wird das Reaktionsgemisch auf Eis gegossen und mit Chloroform extrahiert. Die vereinigten Chloroformextrakte werden mit Natriumbicarbonat neutral gewaschen, über Natriumsulfat getrocknet und im Vakuum eingedampft. Der Rückstand wird in Aceton gelöst und mit einer Lösung von 40 g p-Toluolsulfonsäure in Aceton versetzt. Der ausfallende Niederschlag wird abgesaugt und mit verdünnter Natronlauge/Chloroform geführt. Die Chloroformphase wird abgetrennt, über Natriumsulfat getrocknet und im Vakuum eingedampft. Der Rückstand wird aus Isopropanol umkristallisiert. Man erhält 7-Chlor-1-methyl-2-cyanomethylen-5-(2'-chlorphenyl)-1,3-dihydro-2H-1,4-benzodiazepin vom Schmelzpunkt 162—165°C.

IR-Spektrum (KBr): 2193 $cm^{-1}$ (C=CH—CN).

b) 7-Chlor-1-methyl-2-carbamoylmethylen-5-(2'-chlorphenyl)-1,3-dihydro-2H-1,4-benzodiazepin

5,4 g 7-Chlor-1-methyl-2-cyanomethylen-5-(2'-chlrophenyl)-1,3-dihydro-2H-1,4-benzodiazepin werden portionsweise in 26 ml konzentrierte Schwefelsäure eingetragen und unter allmählicher Erwärmung auf 50°C gelöst. Die Reaktionslösung wird noch 1 Stunde bei 50°C gehalten. Nach dem Abkühlen wird sie zur Aufarbeitung auf Eis gegossen, die Schwefelsäure mit verdünnter Natronlauge neutralisiert und das Rohprodukt mit Methylenchlorid extrahiert. Die organische Phase wird abgetrennt, mit Wasser neutral gewaschen und über Natriumsulfat getrocknet. Nach dem Filtrieren und Eindampfen im Vakuum erhält man einen Rückstand, der durch Anreiben mit Äther kristallisiert. Man erhält 7-Chlor-1-methyl-2-carbamoylmethylen-5-(2'-chlorphenyl)-1,3-dihydro-2H-1,4-benzodiazepin, das aus Äthanol umkristallisiert, einen Schmelzpunkt von 212—217°C besitzt.

Summenformel: $C_{18}H_{15}Cl_2N_3O$.

Elementaranalyse:

| | | | | |
|---|---|---|---|---|
| ber. %: | C 60,01 | H 4,19 | N 11,66 | Cl 19,68 |
| gef. %: | C 59,73 | H 4,26 | N 11,42 | Cl 19,43 |

Arbeitet man nach den obigen Beispielen, so kann man auch die folgenden Verbindungen herstellen:

Beispiel 3

Gemäß Beispiel 1, jedoch unter Verwendung von 7-Brom-1-methyl-2-cyanomethyl-5-(2'-chlorphenyl)-2,3-dihydro-1H-1,4-benzodiazepin erhält man

3a) 7-Brom-1-methyl-2-cyanomethylen-5-(2'-chlorphenyl)-1,3-dihydro-2H-1,4-benzodiazepin, Schmelzpunkt 178—179°C sowie

3b) 7-Brom-1-methyl-2-carbamoylmethylen-5-(2'-chlorphenyl)-1,3-dihydro-2H-1,4-benzodiazepin, Schmelzpunkt 110—115°C unter Zersetzung (enthält 3/4 Mol Aceton).

Beispiel 4

Gemäß Beispiel 2, jedoch unter Verwendung von 7-Brom-1-methyl-2-cyanomethyl-5-(2'-fluorphenyl)-2,3-dihydro-1H-1,4-benzodiazepin erhält man

4a) 7-Brom-1-methyl-2-cyanomethylen-5-(2'-fluorphenyl)-1,3-dihydro-2H-1,4-benzodiazepin, Schmelzpunkt 167—168°C sowie

4b) 7-Brom-1-methyl-2-carbamoylmethylen-5-(2'-fluorphenyl)-1,3-dihydro-2H-1,4-benzodiazepin, Schmelzpunkt 154—155°C (enthält 1/2 Mol Aceton).

Beispiel 5

Gemäß Beispiel 2, jedoch unter Verwendung von 7-Chlor-1-methyl-2-cyanomethyl-5-(2'-fluorphenyl)-2,3-dihydro-1H-1,4-benzodiazepin erhält man

5a) 7-Chlor-1-methyl-2-cyanomethylen-5-(2'-fluorphenyl)-1,3-dihydro-2H-1,4-benzodiazepin, Schmelzpunkt 160—162° C sowie
5b) 7-Chlor-1-methyl-2-carbamoylmethylen-5-(2'-fluorphenyl)-1,3-dihydro-2H-1,4-benzodiazepin, Schmelzpunkt 162—163° C (enthält 1/2 Mol $H_2O$ und 1/3 Mol Aceton).

Beispiel 6

Gemäß Beispiel 2, jedoch unter Verwendung von 7-Nitro-1-methyl-2-cyanomethyl-5-(2'-chlorphenyl)-2,3-dihydro-1H-1,4-benzodiazepin erhält man

6a) 7-Nitro-1-methyl-2-cyanomethylen-5-(2'-chlorphenyl)-1,3-dihydro-2H-1,4-benzodiazepin, Schmelzpunkt 83—90° C sowie
6b) 7-Nitro-1-methyl-2-carbamoylmethylen-5-(2'-chlorphenyl)-1,3-dihydro-2H-1,4-benzodiazepin, Schmelzpunkt 211—214° C.

Beispiel 7

Gemäß Beispiel 1, jedoch unter Verwendung von 7-Chlor-1-methyl-2-cyanomethyl-5-(2'-trifluormethylphenyl)-2,3-dihydro-1H-1,4-benzodiazepin erhält man

7a) 7-Chlor-1-methyl-2-cyanomethylen-5-(2'-trifluormethylphenyl)-1,3-dihydro-2H-1,4-benzodiazepin, Schmelzpunkt 125—130° C,
7b) 7-Chlor-1-methyl-2-carbamoylmethylen-5-(2'-trifluormethylphenyl)-1,3-dihydro-2H-1,4-benzodiazepin, Schmelzpunkt 101—105° C.

Beispiel 8

Gemäß Beispiel 1, jedoch unter Verwendung von 7-Brom-1-methyl-2-cyanomethyl-5-(2'-trifluormethylphenyl)-2,3-dihydro-1H-1,4-benzodiazepin erhält man

8a) 7-Brom-1-methyl-2-cyanomethylen-5-(2'-trifluormethylphenyl)-1,3-dihydro-2H-1,4-benzodiazepin, Schmelzpunkt 128—130° C,
8b) 7-Brom-1-methyl-2-carbamoylmethylen-5-(2'-trifluormethylphenyl)-1,3-dihydro-2H-1,4-benzodiazepin, Schmelzpunkt 115—120° C.

Beispiel 9

Man stellt Tabletten in folgender Zusammensetzung her:

| | pro Tablette |
|---|---|
| 7-Chlor-1-methyl-2-carbamoylmethylen-5-phenyl-1,3-dihydro-2H-1,4-benzodiazepin | 10 mg |
| Maisstärke | 60 mg |
| Milchzucker | 130 mg |
| Gelatine (10% Lösung) | 6 mg |

Der Wirkstoff, die Maisstärke und der Milchzucker werden mit einer 10%igen Gelatine-Lösung angedickt. Die Paste wird zerkleinert; das Granulat wird auf ein geeignetes Blech gebracht und bei 45° C getrocknet.

Das getrocknete Granulat wird durch eine Zerkleinerungsmaschine geleitet, in einem Mixer mit folgenden Ingredienzien vermischt:

| | |
|---|---|
| Talkum | 5 mg |
| Magnesiumstearat | 5 mg |
| Maisstärke | 9 mg |

und sodann zu Tabletten von 225 mg verpreßt.

Beispiel 10

Man stellt Suppositorien mit den folgenden Bestandteilen her:

| | pro 2 g Suppositorium |
|---|---|
| 7-Chlor-1-methyl-2-carbamoylmethylen-5-phenyl-1,3-dihydro-2H-1,4-benzodiazepin | 10 mg |
| Kakaobutter | 1990 mg |

Der Wirkstoff und die fein geriebene Suppositoriengrundmasse werden gründlich gemischt und dann geschmolzen. Aus der durch Rühren homogen gehaltenen Schmelze werden Suppositorien von 2 g gegossen.

Beispiel 11

Eine parenterale Gebrauchsform wird mit den folgenden Bestandteilen hergestellt:

| | pro ml |
|---|---|
| 7-Chlor-1-methyl-2-carbamoylmethylen-5-phenyl-1,3-dihydro-2H-1,4-benzodiazepin | 5 mg |
| Dimethylacetamid | 10% |
| Propylenglykol | 50% |
| Benzylalkohol | 1,5% |
| Äthanol | 10% |
| Wasser für Injektionszwecke, ad | 1 ml |

Der Wirkstoff wird in Dimethylacetamid gelöst und mit Benzylalkohol, Propylenglykol, Äthanol und Wasser versetzt. Man filtriert durch ein Kerzenfilter, füllt in geeignete Ampullen, verschließt und sterilisiert.

Beispiel 12

Man stellt nach den Angaben in den Beispielen 9 bis 11 Tabletten, Suppositoren bzw. eine parenterale Gebrauchsform her, wobei man als Wirkstoff 7-Chlor-1-methyl-2-carbamoylmethylen-5-(2'-chlorphenyl)-1,3-dihydro-2H-1,4-benzodiazepin verwendet.

**Patentansprüche**

1. 1,4-Benzodiazepinderivate der Formel (I)

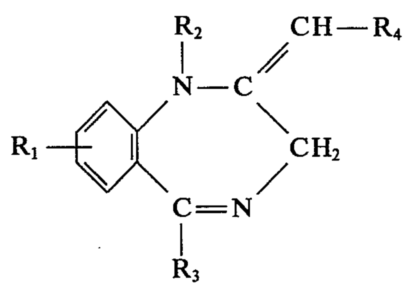

(I)

in welcher

$R_1$ Wasserstoff, Halogen oder Nitro,
$R_2$ Alkyl mit 1–6 Kohlenstoffatomen,
$R_3$ Phenyl, Halogenphenyl oder Trifluormethylphenyl und
$R_4$ Cyano oder Carbamoyl bedeuten,

sowie deren Säureadditionssalze.

2. Verbindungen gemäß Anspruch 1, dadurch gekennzeichnet, daß $R_1$ in 7-Stellung des

Benzodiazepingerüstes steht und Chlor, Brom oder Nitro bedeutet.

3. Verbindungen gemäß den Ansprüchen 1 und 2, dadurch gekennzeichnet, daß $R_2$ für Methyl steht.

4. Verbindungen gemäß den Ansprüchen 1 bis 3, dadurch gekennzeichnet, daß $R_3$ für Phenyl, o-Chlorphenyl, o-Fluorphenyl oder o-Trifluormethylphenyl steht.

5. Verbindungen gemäß den Ansprüchen 1 bis 4, dadurch gekennzeichnet, daß $R_4$ Cyano bedeutet.

6. Verbindungen gemäß den Ansprüchen 1 bis 4, dadurch gekennzeichnet, daß $R_4$ Carbamoyl bedeutet.

7. Verbindungen gemäß Anspruch 1, dadurch gekennzeichnet, daß $R_1$ 7-Chlor, $R_2$ Methyl, $R_3$ Phenyl, o-Chlorphenyl, o-Fluorphenyl oder o-Trifluormethylphenyl und $R_4$ Carbamoyl bedeuten.

8. Verbindungen gemäß Anspruch 1, dadurch gekennzeichnet, daß $R_1$ 7-Brom, $R_2$ Methyl, $R_3$ o-Chlorphenyl, o-Fluorphenyl oder o-Trifluormethylphenyl und $R_4$ Carbamoyl bedeuten.

9. 7-Nitro-1-methyl-2-carbamoylmethylen-5-(2′-chlorphenyl)-1,3-dihydro-2H-1,4-benzodiazepin.

10. Verbindungen der allgemeinen Formel I gemäß Anspruch 1, in denen $R_4$ Carbamoyl bedeutet und $R_1$, $R_2$ und $R_3$ die in Anspruch 1 angegebene Bedeutung besitzen, oder deren pharmazeutisch annehmbare Säureadditionssalze zur Anwendung als Psychosedativa.

11. Verfahren zur Herstellung von 1,4-Benzodiazepinderivaten der Formel (I)

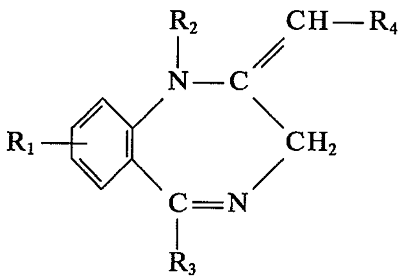

(I)

in welcher

$R_1$ Wasserstoff, Halogen oder Nitro,
$R_2$ Alkyl mit 1–6 Kohlenstoffatomen,
$R_3$ Phenyl, Halogenphenyl oder Trifluormethylphenyl und
$R_4$ Cyano oder Carbamoyl bedeuten,

sowie deren Säureadditionssalze, dadurch gekennzeichnet, daß man eine Verbindung der Formel (II)

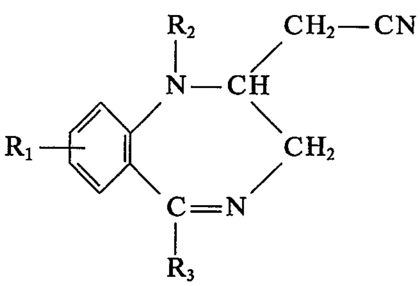

(II)

in welcher $R_1$, $R_2$ und $R_3$ die oben angegebene Bedeutung haben, in stark saurer Lösung mit Chrom(VI)-oxid in gelöster oder feinsuspendierter Form zu einer Verbindung der Formel (Ia)

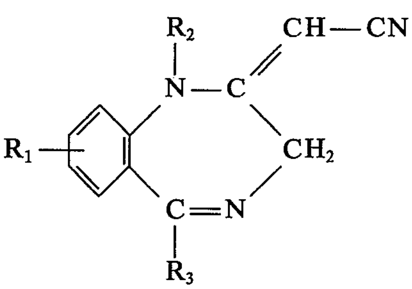

(Ia)

in welcher $R_1$, $R_2$ und $R_3$ die oben angegebene Bedeutung haben, oxydiert und gegebenenfalls durch saure Hydrolyse des Cyanorestes zum Carbamoylrest in die Verbindung der Formel (Ib)

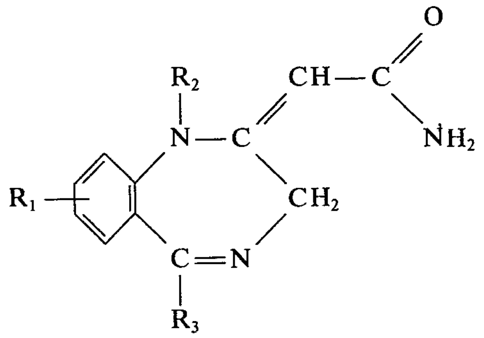

(Ib)

in welcher $R_1$, $R_2$ und $R_3$ die oben angegebene Bedeutung haben, überführt sowie die erhaltenen freien Basen gegebenenfalls in die Säureadditionssalze bzw. die erhaltenen Säureadditionssalze in die freien Basen überführt.

12. Arzneimittel, die neben üblichen Hilfs- und Trägerstoffen mindestens ein 1,4-Benzodiazepinderivat der allgemeinen Formel (I) gemäß Anspruch 1, in der $R_1$, $R_2$ und $R_3$ die dort angegebene Bedeutung haben und $R_4$ Carbamoyl ist oder ein pharmazeutisch annehmbares Säureadditionssalz davon, enthalten.

## Claims

1. 1,4-Benzodiazepine derivatives of the formula (I)

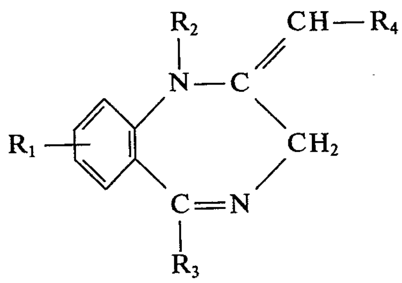

(I)

in which

$R_1$ is hydrogen, halogen or nitro,
$R_2$ is alkyl with 1 to 6 carbon atoms,
$R_3$ is phenyl, halophenyl or trifluoromethylphenyl and
$R_4$ is cyano or carbamoyl,

and their acid addition salts.

2. Compounds according to Claim 1, characterised in that $R_1$ is in 7-position of the benzodiazepine structure and is chlorine, bromine or nitro.

3. Compounds according to Claims 1 and 2, characterised in that $R_2$ stands for methyl.

4. Compounds according to Claims 1 to 3, characterised in that $R_3$ stands for phenyl, o-chlorophenyl, o-fluorophenyl or o-trifluoromethyl phenyl.

5. Compounds according to Claims 1 to 4, characterised in that $R_4$ is cyano.

6. Compounds according to Claims 1 to 4, characterised in that $R_4$ is carbamoyl.

7. Compounds according to Claim 1, characterised in that $R_1$ is 7-chloro, $R_2$ is methyl, $R_3$ is phenyl, o-chlorophenyl, o-fluorophenyl or o-trifluoromethylphenyl and $R_4$ is carbamoyl.

8. Compounds according to Claim 1, characterised in that $R_1$ is 7-bromo, $R_2$ is methyl, $R_3$ is o-chlorophenyl, o-fluorophenyl or o-trifluoromethylphenyl and $R_4$ is carbamoyl.

9. 7-nitro-1-methyl-2-carbamoylmethylene-5-(2′-chlorophenyl)-1,3-dihydro-2H-1,4-benzodiazepine.

10. Compounds of the general Formula I according to Claim 1, in which $R_4$ is carbamoyl and $R_1$, $R_2$ and $R_3$ have the meanings indicated in Claim 1, or their pharmaceutically acceptable acid addition salts for use as psychosedatives.

11. Method for the production of 1,4-benzodiazepine derivatives of the formula (I)

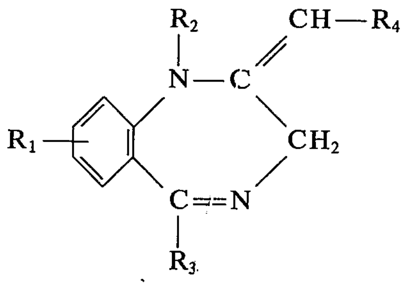

(I)

in which

$R_1$ is hydrogen, halogen or nitro,
$R_2$ is alkyl with 1 to 6 carbon atoms,
$R_3$ is phenyl, halophenyl or trifluoromethylphenyl and
$R_4$ is cyano or carbamoyl,

and their acid addition salts, characterised in that a compound of the formula (II)

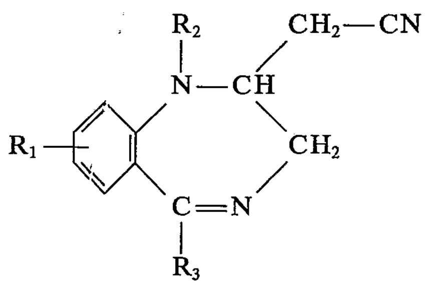

(II)

in which $R_1$, $R_2$ and $R_3$ have the meanings indicated above, is oxidized, in a strongly acidic solution with chrom(VI)-oxide, in dissolved or finely suspended form, to a compound of the formula (Ia)

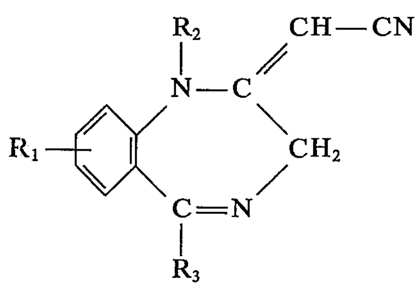

(Ia)

in which $R_1$, $R_2$ and $R_3$ have the meanings indicated above, and, if required, through acidic hydrolysis of the cyano radical to the carbamoyl radical, is converted into the compound of the formula (Ib)

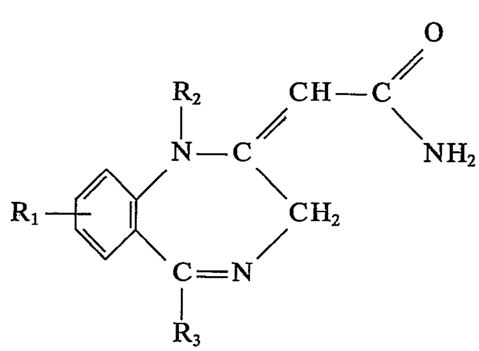

(Ib)

in which $R_1$, $R_2$ and $R_3$ have the meanings indicated above, and the free bases obtained, if required, are converted, are converted into the acid addition salts or the acid addition salts obtained are converted

into the free bases.

12. Pharmaceutical preparations, which in addition to the usual adjuvants and carrier substances contain at least one 1,4-benzodiazepine derivative of the general formula (I) according to Claim 1, in which $R_1$, $R_2$ and $R_3$ have the meanings indicated therein, and $R_4$ is carbamoyl or a pharmeceutically acceptable acid addition salt thereof.

**Revendications**

1. Dérivés de 1,4-benzodiazépines de formule (I):

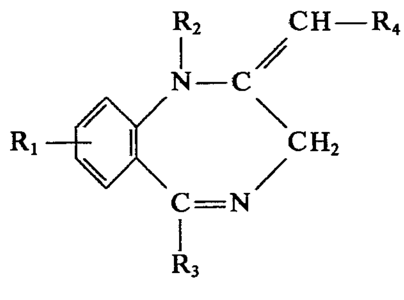

(I)

dans laquelle

$R_1$ représente un atome d'hydrogène, un atome d'halogène ou un groupe nitro,
$R_2$ représente un groupe alkyle contenant 1 à 6 atomes de carbone,
$R_3$ représente un groupe phényle, un groupe halogénophényle ou un groupe trifluorométhylphényle, et
$R_4$ représente un groupe cyano ou un groupe carbamoyle,

de même que leurs sels d'addition d'acide.

2. Composés suivant la revendication 1, caractérisés en ce que $R_1$ occupe la position 7 du squelette de benzodiazépine et représente un atome de chlore, un atome de brome ou un groupe nitro.

3. Composés suivant les revendications 1 et 2, caractérisés en ce que $R_2$ représente un groupe méthyle.

4. Composés suivant les revendications 1 à 3, caractérisés en ce que $R_3$ représente un groupe phényle, un groupe o-chlorophényle, un groupe o-fluorophényle ou un groupe o-trifluorométhylphényle.

5. Composés suivant les revendications 1 à 4, caractérisés en ce que $R_4$ représente un groupe cyano.

6. Composés suivant les revendications 1 à 4, caractérisés en ce que $R_4$ représente un groupe carbamoyle.

7. Composés suivant la revendication 1, caractérisés en ce que $R_1$ représente un atome 7-chloro, $R_2$ représente un groupe méthyle, $R_3$ représente un groupe phényle, un groupe o-chlorophényle, un groupe o-fluorophényle ou un groupe o-trifluorométhylphényle et $R_4$ représente un groupe carbamoyle.

8. Composés suivant la revendication 1, caractérisés en ce que $R_1$ représente un atome 7-bromo, $R_2$ représente un groupe méthyle, $R_3$ représente un groupe o-chlorophényle, un groupe o-fluorophényle ou un groupe o-trifluorométhylphényle et $R_4$ représente un groupe carbamoyle.

9. La 7-nitro-1-méthyl-2-carbamoylméthylène-5-(2'-chlorophényl)-1,3-dihydro-2H-1,4-benzodiazépine.

10. Composés répondant à la formule générale I suivant la revendication 1 dans laquelle $R_4$ représente un groupe carbamoyle, tandis que $R_1$, $R_2$ et $R_3$ ont les significations indiquées dans la revendication 1, ou leurs sels d'addition d'acide pharmaceutiquement acceptables en vue de les utiliser comme psychosédatifs.

11. Procédé de préparation de dérivés de 1,4-benzodiazépines de formule (I):

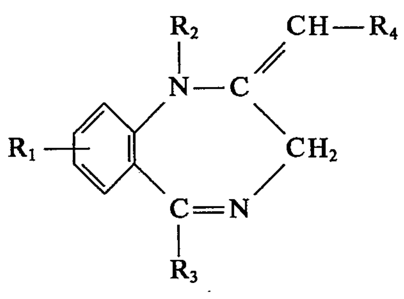

(I)

dans laquelle

$R_1$ représente un atome d'hydrogène, un atome d'halogène ou un groupe nitro,
$R_2$ représente un groupe alkyle contenant 1 à 6 atomes de carbone,
$R_3$ représente un groupe phényle, un groupe halogénophényle ou un groupe trifluorométhylphényle, et
$R_4$ représente un groupe cyano ou un groupe carbamoyle,

ainsi que de leurs sels d'addition d'acide, caractérisé en ce qu'on oxyde un composé de formule (II):

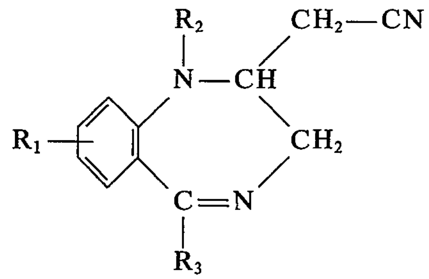

(II)

dans laquelle $R_1$, $R_2$ et $R_3$ ont les significations indiquées ci-dessus, dans une solution fortement acide avec de l'oxyde de chrome(VI) sous forme d'une solution ou d'une fine suspension, pour obtenir un composé de formule (Ia):

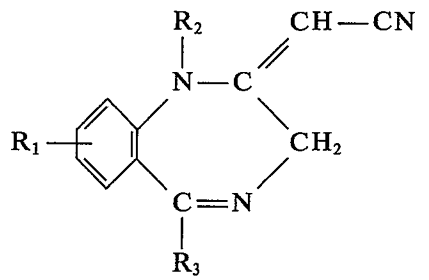

(Ia)

dans laquelle $R_1$, $R_2$ et $R_3$ ont les significations indiquées ci-dessus, et on transforme éventuellement ce composé en un composé de formule (Ib)

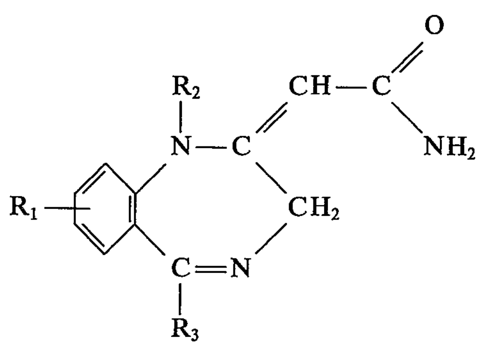

(Ib)

dans laquelle $R_1$, $R_2$ et $R_3$ ont les significations indiquées ci-dessus, par hydrolyse acide du groupe cyano en un groupe carbamoyle, tandis que l'on transforme éventuellement les bases libres obtenues en sels d'addition d'acide et les sels d'addition d'acide obtenus, en bases libres.

12. Médicaments qui, outre des substances auxiliaires et des substances supports habituelles, contiennent au moins un dérivé de 1,4-benzodiazépine de formule générale (I) suivant la revendication 1, dans laquelle $R_1$, $R_2$ et $R_3$ ont les significations y indiquées, tandis que $R_4$ représente un groupe carbamoyle, ou encore un sel d'addition d'acide pharmaceutiquement acceptable de ce dérivé.